# EUROPEAN PATENT SPECIFICATION

(11) **EP 4 163 386 B1**
(45) Date of publication and mention of the grant of the patent: **19.08.2026**
(21) Application number: 20939371.9
(22) Date of filing: 29.09.2020
(51) Int. Cl.: C12Q 1/6886

(54) **GENETIC MARKER COMBINATION AND APPLICATION THEREOF**
GENETISCHE MARKERKOMBINATION UND ANWENDUNG DAVON
COMBINAISON DE MARQUEUR GÉNÉTIQUE ET APPLICATION ASSOCIÉE

(30) Priority: 03.06.2020 CN 202010494245
(43) Date of publication of application: 12.04.2023
(73) Proprietor: Creative Biosciences (Guangzhou) Co., Ltd., Guangzhou, Guangdong 510555 (CN)
(72) Inventor: WU, Xiaolin, Guangzhou, Guangdong 510530 (CN); LI, Shiliang, Guangzhou, Guangdong 510530 (CN); ZHANG, Zhiwei, Guangzhou, Guangdong 510530 (CN); CHEN, Xinzhou, Guangzhou, Guangdong 510530 (CN); WU, Youzhi, Guangzhou, Guangdong 510530 (CN); GU, Yunjuan, Guangzhou, Guangdong 510530 (CN); ZOU, Hongzhi, Guangzhou, Guangdong 510530 (CN)
(74) Representative: J A Kemp LLP
(86) International application number: PCT/CN2020/118998
(87) International publication number: WO 2021/243904

(56) References cited:
- EP-A1- 2 966 183
- WO-A1-2021/243906
- CN-A- 103 463 621
- CN-A- 104 520 442
- CN-A- 107 922 941
- CN-A- 110 998 319
- CN-A- 111 182 924
- CN-A- 111 676 287
- US-A1- 2017 137 871
- OOKI AKIRA ET AL: "A Panel of Novel Detection and Prognostic Methylated DNA Markers in Primary Non-Small Cell Lung Cancer and Serum DNA", CANCER RESEARCH, vol. 23, no. 22, 29 August 2017 (2017-08-29), US, pages 7141 - 7152, XP055851310, ISSN: 1078-0432, DOI: 10.1158/1078-0432.CCR-17-1222
- DONG JINGSI ET AL: "LincRNA00494 Suppresses Non-small Cell Lung Cancer Cell Proliferation by Regulating SRCIN1 Expression as a ceRNA", FRONTIERS IN ONCOLOGY, vol. 10, no. 79, 6 February 2020 (2020-02-06), pages 1 - 8, XP055876566, DOI: 10.3389/fonc.2020.00079

## Description

### FIELD

The present disclosure relates to the field of biological medicine, and particularly relates to a combination of gene markers and use thereof.

### BACKGROUND

Lung cancer is a malignant lung tumor derived from tunica mucosa bronchiorum, glands or alveolar epithelium. According to pathological patterns, lung cancer can be divided into: 1) small cell lung cancer (SCLC): SCLC is a lung cancer of a special pathological type and has obvious distant metastasis propensity and poor prognosis, but most of the patients are sensitive to chemoradiotherapy; 2) non-small cell lung cancer (NSCLC): the NSCLC is a lung cancer of other pathological types except for the SCLC and includes squamous-cell carcinoma, adenomatous carcinoma and large cell carcinoma. Lung cancer has a certain difference in aspects of biological behavior and clinical course. According to occurrence location, lung cancer can also be divided into: 1) central lung cancer: a lung cancer growing at the segmental bronchi opening and above; and 2) peripheral lung cancer: a lung cancer growing beyond the segmental bronchi opening.

In recent years, due to the effects of factors such as population aging, atmospheric contamination and smoking, morbidity and mortality of lung cancer are increased progressively year by year in China. China Cancer Registry Annual Report (2017*)* shows that about 7 persons are diagnosed with cancers every minute throughout China, wherein the morbidity and mortality of lung cancer take the first place. China has become the country with the largest number of people suffering from lung cancer in the world. Experts forecast that the number of people suffering from lung cancer will be up to 1 million by 2025. Further, according to the epidemiologic study, smoking is an important factor in causing lung cancer. About 80-90% of lung cancer can be attributed to smoking in the world. Compared with nonsmokers, people at an age of 45-64 who smoke 1-19 cigarettes and 20 cigarettes or more per day have respective relative risk (4.27 and 8.61) of suffering from lung cancer; and compared with people who had never smoked, people who smoke 1-19 cigarettes and 20 cigarettes or more per day for a long time have relative risk (6.14 and 10.73) of dying of the lung cancer. Although treatment technology for lung cancer changes quickly, a survival rate of 5 years is only increased from 4% to about 12%. Existing antitumor drugs can still only achieve the effect of alleviating the disease, and progression-free survival of the patients is averagely prolonged by 3-5 months only. However, postoperative phase-I lung cancer patients have a 5-year survival rate of up to about 60-70%. Therefore, early diagnosis and early operation of lung cancer are one of the most effective methods for increasing the 5-year survival rate of lung cancer and decreasing mortality.

At present, there are several major clinical auxiliary diagnosis methods for lung cancer, but cannot completely realize early discovery and early diagnosis of lung cancer.
(1) Biochemical blood examination: there is no specific biochemical blood examination for primary lung cancer currently. Elevation of alkaline phosphatase or blood calcium in the blood of lung cancer patients should consider the possibility of bone metastasis, while the elevation of alkaline phosphatase, glutamic oxalacetic transaminase, lactic dehydrogenase or bilirubin in the blood should consider the possibility of hepatic metastasis.
(2) Tumor marker examination: 1) CEA: abnormally high-level CEA exists in the serum of 30-70% of lung cancer patients, but mainly in advanced lung cancer patients. At present, examination of the CEA in serum is mainly used for estimating the prognosis of lung cancer and monitoring the treatment process. 2) NSE: the NSE is a preferred marker for small cell lung cancer, is used for diagnosing small cell lung cancer and monitoring therapeutic response, and has different reference values according to differences in test methods and used reagents. 3) CYFRA21-1: the CYFRA21-1 is a preferred marker for non-small cell lung cancer, can achieve a sensitivity of 60% to a diagnosis of squamous cell lung carcinoma, and has different reference values according to differences in test methods and used reagents.
(3) Imageological examination: 1) chest X-ray examination: the chest X-ray examination should include chest PA and lateral projection. In a primary hospital, chest PA and lateral projection is still the most basic and preferred imaging diagnosis method during the preliminary diagnosis of lung cancer. Once lung cancer is diagnosed or suspected, a chest CT examination is conducted. 2) CT examination: the chest CT is the most common and important examination method for lung cancer and is used for diagnosis and differential diagnosis of lung cancer and staging and follow-up examination after treatment. CT-guided transthoracic needle biopsy is an important diagnostic technique for lung cancer. In hospitals with good conditions, the CT-guided transthoracic needle biopsy can be used for the diagnosis of difficult qualitative pulmonary lesions, and clinical diagnosis of lung cancer needs cytological and histological confirmation when materials are difficult to be drawn in other methods. In recent years, multi-slice spiral CT and low-dose CT (LDCT) are effective screening tools for discovering early lung cancer and lowering mortality. American National Lung Cancer Screening Test (NLST) has shown that, compared with chest X-ray screening, the LDCT can lower the mortality of lung cancer by 20%. Low-dose spiral CT is recommended as an important means of screening early lung cancer. However, due to more human influence factors, a false positive rate is extremely high. 3) Ultrasonic testing: ultrasonic testing is mainly used for discovering whether abdominal vital organs and abdominal and retroperitoneal lymph nodes have metastasis and can also be used for examination of cervical lymph nodes. Solid-cystic characteristics of pulmonary lesions adjacent to the chest walls or chest wall lesions can be identified, and ultrasound-guided percutaneous biopsy is conducted. Ultrasonic testing is further often used for hydrothorax extraction and location. 4) Bone scanning: bone scanning has higher sensitivity to bone metastasis detection of lung cancer but has a certain false positive rate. Bone scanning can be used for the following conditions: a preoperative examination of lung cancer; patients with local symptoms.
(4) Other examinations: 1) sputum cytology examination: the sputum cytology examination is a simple and convenient noninvasive diagnosis method for lung cancer currently, can increase a positive rate by about 60% through continuous smear examination, and is a routine diagnostic method for suspected lung cancer cases. 2) fiberoptic bronchoscopy: the fiberoptic bronchoscopy is one of the most important means in lung cancer diagnosis, plays an important role in qualitative location diagnosis of the lung cancer and operation plan selection, and is a necessary routine examination item for patients to be subjected to operative treatment. However, although transbronchial needle aspiration (TBNA) is favorable for staging before treatment, due to higher technical difficulty and risk, people in need may transfer to a higher-level hospital for further examination. 3) Others: the other examinations include percutaneous lung biopsy, video-assisted thoracic surgery, mediastinal biopsy, hydrothorax cytology examination and can be respectively used for assisting diagnosis according to existing conditions in presence of indication.

The multi-slice spiral CT and the low-dose CT (LDCT) in imageological examination are effective screening tools for discovering early lung cancer and lowering mortality. American National Lung Cancer Screening Test (NLST) has shown that, compared with chest X-ray screening, the LDCT can lower the mortality of lung cancer by 20%. It is proved in clinical practice work that, the success or failure of any lung cancer screening item depends on the identification of a high-risk group. A risk prediction model fused with multiple high-risk factors has been acknowledged in the world as one of the methods for identifying the high-risk group of lung cancer. By assisting clinicians in the improvement of intervening measures or treatment means, the risk model further improves the curative effects of lung cancer patients. Although it is acknowledged in the world that screening specified at the high-risk group can lower currently higher mortality of lung cancer, the definition of the high-risk group is still a problem difficult to solve. To maximize a benefit-injury ratio of lung cancer screening, the first key problem is how to define a group at high disease risk; and the second key problem is to determine a method for screening the group, including the definition of high-risk factors, quantitative summarization of total risk and selection of a screening benefit margin.

With the rapid development of technology, tumor marker detection becomes a new field of tumor diagnosis and treatment following the imaging diagnosis and pathological diagnosis and can achieve a significant impact on the diagnosis, detection, and treatment of tumors. The tumor marker may be detected in body fluids or tissues and can reflect the existence of the tumors, differentiated degrees, prognosis estimation, personalized medication, therapeutic effects, and the like. Early lung cancer patients have no obvious symptoms; early lung cancer is difficult to perceive by doctors and patients; and since there is no obvious specific marker in the blood or biochemical items, early discovery and early diagnosis are difficult to be conducted through a routine diagnosis method. Therefore, early diagnosis of lung cancer, particularly the screening of a large-scale application population, is relatively difficult.

More and more researches show that two major categories of mechanisms are included in a tumor formation process. One mechanism forms mutation through DNA nucleotide sequence change and is called a genetic mechanism. The tumor serving as a genetic disease has been proven in the field of molecular biology. The other mechanism is an epigenetics mechanism and can change a gene expression level independent of the DNA sequence change, and effects of the epigenetics mechanism get more and more attention in the tumor formation process. The genetic mechanism and the epigenetics mechanism are in cross existence and promote the formation of the tumors together. Aberrant methylation of genes may occur at an early stage of tumor occurrence; and in a gradual development process of the tumors, an aberrant methylation degree of the genes is increased. Through analysis of genome of 98 common human primary tumors, it is discovered that each tumor has at least includes 600 aberrantly methylated CpG islands.

Many studies have shown that aberrant methylation of a promoter is a frequent early event in the occurrence process of many tumors. Therefore, the methylation status of tumor-related genes is an early sensitive indicator of tumorigenesis and is identified as a prospective tumor molecule biomarker. More importantly, cancerization cells may release DNA into the peripheral blood. Nanogram-level free DNA exists in the peripheral blood of a normal person. The study finds that aberrant promoter methylation of tumor-related genes existing in tumor tissues can also be detected in peripheral blood plasma/serum, tumor involvement, and organ-related body fluid (such as saliva and sputum). These biological samples are easily available and can be sensitively detected by conducting amplifying lots of DNA in the samples through PCR technology. Therefore, detecting a methylation status of a promoter region of some tumor-related genes can provide very valuable information for early diagnosis of the tumors. Compared with tumor molecule markers of other types, the biomarker has more advantages in the detection of the aberrant methylation of the promoter. A certain gene has the same region of the aberrant methylation of the promoter in different types of tumors, and then detection is relatively convenient. In addition, compared with an allele deletion marker, the aberrant methylation is a positive signal and is easily distinguished from a negative background in normal tissues. Esteller *et al.* detect the aberrant methylation status of the promoter region of genes such as p16, DAPK, GSTP1, and MGM T in tumor tissues and serum of 22 NSCLC cases, and discover that promoter methylation of at least one gene exists in 68% (15/22) of the tumor tissues. However, in 15 positive tissue cases, the existence of the aberrant methylation of the promoter is also detected in the serum of 11 cases. Additionally, many researchers detect the promoter methylation of some tumor-related genes from tumor tissues and serum of patients with liver cancer, head-neck carcinoma, esophagus cancer, and colon cancer respectively.

The existing lung cancer detection technology is mainly low in sensitivity, high in false positive rate, and invasive; and early lung cancer is difficult to be detected by the current routine detection technology.

Further, non-invasive detection, such as sputum detection, of lung cancer has higher difficulty. Although a tumor marker in the sputum of lung cancer patients is researched by researchers, compared with the detection and evaluation of the tumor marker in blood samples of other tumor patients, the success rate of the sputum samples is extremely low. Major reasons are as follows: (1) the composition of the sputum is complex, and different groups have greater differences in components and viscosity of the sputum in different diseases or environments; (2) the sputum contains components of many bronchial epithelial cells and bacteria, oral mucosa cells and other non-lung cancer cells; and sufficient DNA derived from the lung cancer cannot be effectively enriched by a general sample treatment method; (3) many smoking patients have no expectoration. Studies of A. J. Hubers et al. in Molecular Sputum Analysis for the Diagnosis of Lung Cancer to the 10 previous papers show that a methylation degree of the median of the marker in the lung cancer tissues is 48%, while the methylation degree of the median in the sputum is 38%. The results show that the detection rate of the methylation marker in the tissues is significantly higher than that in the sputum. Meanwhile, Rosalia Cirincione (*Methylation profile in tumor and sputum samples of lung cancer patients detected by spiral computed tomography: A nested case-contro*) reports that the detection rate of RARbeta2, P16, and RASSF1A in the lung cancer tissues is respectively up to 65.5%, 41.4%, and 51.7%, while the detection rate is respectively up to 44.4%, 5% and 5% in the sputum only.

US 2017/0137871 A1 discloses in example 4 that methylation is determined for a promoter region of a gene selected from the group comprising HOXB4 in the context of breast cancer, and also refers to lung cancer in example 16.

EP 2,966,183 A1 discloses a method for obtaining information on lung cancer comprising the steps of: preparing a DNA sample from a biological sample collected from a subject; analysing methylation status of a CpG site located in a promoter region of at least one gene selected from HOXB4 and ZSCAN31 in the DNA sample obtained in the preparing step; and obtaining information on lung cancer in the subject based on an analysis result obtained in the analysing step (cf. claim 1).

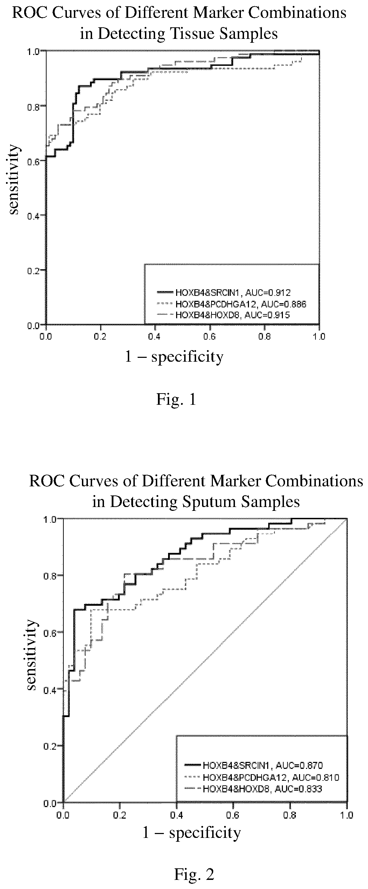

Akira Ooki et al., Cancer Res. 2017, Vol. 23, No. 22, pp. 7141-7152 discloses HOXB4 in figure 1 as putative prognostic lung cancer marker.

Jingsi Dong et al., Front. Oncol. 2020, Vol. 10, No. 79, pp. 1-8 discloses the possible involvement of SRCIN1 in lung cancer.

### SUMMARY

The invention is set out in the appended set of claims.

One of the objects of the present disclosure is to provide a combination of gene markers, as well as a detection/diagnostic reagent for detecting the combination of gene markers and a use of the combination of gene markers.

In one aspect, the present disclosure provides a combination of gene markers. The combination of gene markers includes HOXB4 and SRCIN1.

The combination of gene markers in the present disclosure further includes a fragment of any length in each gene marker. In other words, a combination of any fragment from the HOXB4 and any fragment from SRCIN1 (the fragment may be of any length) falls within the protection scope of the present disclosure.

HOXB4 gene is a member of an Antp homeobox gene family and belongs to homeobox cluster B genes on Chromosome 17. A nucleoprotein with a homeobox DNA binding domain is coded, and the coded protein serves as a specific sequence transcription factor participating in development. Intracellular or ectopic expression of the protein may amplify hematopoietic stem cells and progenitor cells *in vivo* and *in vitro* so that the protein becomes a potential candidate for therapeutic stem cell amplification.

SRCIN1 has the full name of SRC kinase signaling inhibitor 1. The gene and protein of SRCIN1 serving as a negative regulatory factor of SRC inhibit SRC activity and downstream signal transduction by activating CSK, thereby causing cell diffusion and migration impairment. The SRCIN1 regulates the morphology of dendritic spines and participates in calcium-dependent exocytosis.

The present disclosure further provides a use of a multi-gene combined methylation detection reagent in the preparation of a lung cancer detection reagent or a kit. The genes include HOXB4 and SRCIN1.

At present, the missing detection rate of lung cancer is higher. Particularly, non-invasive sputum detection for the type of adenomatous carcinoma is extremely difficult, and the detection rate is extremely low. The reason is as follows: most of the adenomatous carcinoma originates from smaller bronchus and is peripheral lung cancer, and cast-off cells deep into the lung are difficult to be coughed out through sputum. Therefore, there is almost zero sputum detection means for adenomatous carcinoma at present.

Lowering the missing detection rate is particularly important in early tumor screening. If an early tumor screening product cannot screen the total or the vast majority of patients, patients who are not detected will not get enough risk warnings, and the treatment opportunity will be delayed, which is a massive loss for the patients.

Although some lung cancer-related tumor markers have been discovered in the prior art, limited by detection reagents or detection means of these tumor markers, requirements on sensitivity and specificity of these tumor markers cannot be met. Therefore, at present, screening means that can be practically applied to lung cancer still needs to be further researched in the art. However, although non-invasive screening has unique advantages in sampling, the non-invasive screening also has some limitations in other aspects. For example, for the type of adenomatous carcinoma in lung cancer, since the cast-off cells deep into the lung are difficult to be coughed out through the sputum, generally those skilled in the art will consider that lung cancer of the type is unsuitable for the non-invasive screening. Further, even if lung cancer of any other type, a non-invasive screening method that has been reported so far is difficult to meet clinical application requirements. Although related research has been conducted for many years, there is still no non-invasive screening method for lung cancer that can be clinically applied up to now.

The present disclosure further provides a multi-gene combined methylation detection reagent or kit, including a methylation detection reagent for HOXB4 and SRCIN1 genes.

The "methylation detection reagent" includes the following contents: a detection reagent specified at any smaller/shorter sequence of the gene or gene contents. In other words, detection conducted specified at any locus in the gene (*e.g.,* a smaller fragment) and the detection reagent should fall within the protection scope of the present disclosure.

The gene markers HOXB4 and SRCIN1 in the present disclosure are detected in a combined manner. In other words, the several gene markers in the present disclosure are detected simultaneously.

The "detection" in the present disclosure means diagnosis, includes middle- and late-stage diagnosis of the lung cancer in addition to early diagnosis of lung cancer and further includes lung cancer screening, risk evaluation, prognosis, disease identification, diagnosis of disease stages, and selection of therapeutic targets.

Due to the application of the lung cancer marker combination HOXB4 and SRCIN1, early diagnosis of lung cancer becomes possible. When it is determined that methylated genes in cancer cells are methylated in clinically or morphologically normal appearance cells, it is indicated that the normal appearance cells develop into cancer. Thus, lung cancer can be diagnosed through methylation of the lung cancer-specific HOXB4 and SRCIN1 gene combination in the normal appearance cells at an early stage.

The early diagnosis includes a discovery of cancer possibility before metastasis, preferably before the observation of morphological changes in tissues or cells.

In addition to the early diagnosis of lung cancer, the reagent/kit in the present disclosure is expected to be used for lung cancer screening, risk evaluation, prognosis, disease identification, diagnosis of disease stages, and selection of therapeutic targets.

As an optional embodiment of the disease stages, lung cancer can be diagnosed by measuring the methylation of the HOXB4 and SRCIN1 gene combination obtained from the samples by virtue of the progress of the lung cancer at different stages or phases. By comparing a methylation degree of the HOXB4 and SRCIN1 gene combination of nucleic acids isolated from samples at each stage of the lung cancer with a methylation degree of the HOXB4 and SRCIN1 gene combination of one or more nucleic acids isolated from samples in tissues without cell proliferation anomaly, a specific stage of the lung cancer in the samples can be detected.

Generally, CpG islands refer to some regions enriched in CpG dinucleotides and are generally located at the promoter and a nearby region. The CpG island in the present disclosure not only refers to the CpG dinucleotide enriched in the promoter and the nearby region, but also includes a CpG locus with heterozygous methylation, or an isolated CpG locus.

The combined methylation detection reagent in the HOXB4 and SRCIN1 gene combination can be a methylation detection reagent in the prior art. In the prior art, methylation of target genes can be detected by multiple existing methods, such as methylation-specific PCR (MSP), quantitative methylation-specific PCR (qMSP), methylation DNA specific binding protein PCR, quantitative PCR and DNA chip, methylation-sensitive restriction endonucleases, bisulfite sequencing or pyrosequencing. In addition, other methylation detection methods may be introduced through US provisional application 62/007,687. Each detection method includes a corresponding reagent. These reagents can be all used for detecting the methylation of the HOXB4 and SRCIN1 gene combination in the present disclosure.

The present disclosure further provides a combined methylation detection reagent for the HOXB4 and SRCIN1 gene combination. The combined methylation detection reagent includes a primer and/or a probe specified at each gene in the HOXB4 and SRCIN1 gene combination.

In some specific embodiments of the present disclosure, the detection reagent includes a primer and/or a probe obtained from CpG islands of each gene in the HOXB4 and SRCIN1 gene combination.

In some specific embodiments of the present disclosure, the primer and/or the probe detects methylation of each gene in the HOXB4 and SRCIN1 gene combination through quantitative Methylation-Specific PCR (qMSP).

In some specific embodiments of the present disclosure, the methylation detection reagent provided by the present disclosure detects methylation levels of a genosome, an intergenic region, or a promoter region and a region near the promoter region of each gene in the HOXB4 and SRCIN1 gene combination.

In some embodiments, the methylation detection reagent provided by the present disclosure comprises a primer and/or a probe obtained from CpG islands of a promoter region or a region near the promoter region of each gene in the HOXB4 and SRCIN1 gene combination.

In some embodiments, in the methylation detection reagent provided by the present disclosure, a forward primer in the primers for methylation detection of the HOXB4 gene includes any of the nucleotide sequences shown as follows:
I. a nucleotide sequence having an identity of at least 85% or at least 90% or at least 91% or at least 92% or at least 93% or at least 94% or at least 95% or at least 96% or at least 97% or at least 98% or at least 99% or 100% to nucleotide sequences shown as SEQ ID NO: 1, SEQ ID NO: 16 and SEQ ID NO: 19; and
II. a complementary sequence of the sequence as shown in I; and/or
   a reverse primer in the primers for methylation detection of the HOXB4 gene includes any of the nucleotide sequences shown as follows:
III. a nucleotide sequence having an identity of at least 85% or at least 90% or at least 91% or at least 92% or at least 93% or at least 94% or at least 95% or at least 96% or at least 97% or at least 98% or at least 99% or 100% to nucleotide sequences shown as SEQ ID NO: 2, SEQ ID NO: 17 and SEQ ID NO: 20;
IV. a complementary sequence of the sequence as shown in III; and/or
   a forward primer in the primers for methylation detection of the SRCIN1 gene includes any of the nucleotide sequences shown as follows:
V. a nucleotide sequence having an identity of at least 85% or at least 90% or at least 91% or at least 92% or at least 93% or at least 94% or at least 95% or at least 96% or at least 97% or at least 98% or at least 99% or 100% to nucleotide sequences shown as SEQ ID NO: 4 and SEQ ID NO: 22; and
VI. a complementary sequence of the sequence as shown in V; and/or
   a reverse primer in the primers for methylation detection of the SRCIN1 gene includes any of the nucleotide sequences shown as follows:
VII. a nucleotide sequence having an identity of at least 85% or at least 90% or at least 91% or at least 92% or at least 93% or at least 94% or at least 95% or at least 96% or at least 97% or at least 98% or at least 99% or 100% to a nucleotide sequence shown as SEQ ID NO: 5; and
VIII. a complementary sequence of the sequence as shown in VII.

In some embodiments, the primer pair for methylation detection of the HOXB4 gene is shown as SEQ ID NO: 1 and SEQ ID NO: 2.

In some embodiments, the primer pair for methylation detection of the HOXB4 gene is shown as SEQ ID NO: 16 and SEQ ID NO: 17.

The primer pair for methylation detection of the HOXB4 gene is shown as SEQ ID NO: 19 and SEQ ID NO: 20.

In some embodiments, the primer pair for methylation detection of the SRCIN1 gene is shown as SEQ ID NO: 4 and SEQ ID NO: 5.

In some embodiments, the primer pair for methylation detection of the SRCIN1 gene is shown as SEQ ID NO: 22 and SEQ ID NO: 5.

In some embodiments, the probe for methylation detection of the HOXB4 gene has any of the nucleotide sequences shown as follows:
IX. a nucleotide sequence having an identity of at least 85% or at least 90% or at least 91% or at least 92% or at least 93% or at least 94% or at least 95% or at least 96% or at least 97% or at least 98% or at least 99% or 100% to nucleotide sequences shown as SEQ ID NO: 3, SEQ ID NO: 18 and SEQ ID NO: 21; and
X. a complementary sequence of the sequence as shown in IX; and/or
the probe for methylation detection of the SRCIN1 gene has any of the nucleotide sequences shown as follows:
   XI. a nucleotide sequence having an identity of at least 85% or at least 90% or at least 91% or at least 92% or at least 93% or at least 94% or at least 95% or at least 96% or at least 97% or at least 98% or at least 99% or 100% to a nucleotide sequence shown as SEQ ID NO: 6; and
   XII. a complementary sequence of the sequence as shown in XI.

In some embodiments, the lung cancer is selected from small cell lung cancer and non-small cell lung cancer.

In some embodiments, the non-small cell lung cancer is selected from squamous-cell carcinoma or adenomatous carcinoma.

The present disclosure further provides a kit for detecting lung cancer. The kit includes the combined methylation detection reagent.

In some embodiments, the kit provided by the present disclosure further includes common reagents in the kit. For example, a common transforming agent in qMSP is used for transforming a non-methylated cytosine base into uracil, while a methylated cytosine base stays the same. The transforming agent is not specially limited. All reagents that can transform the cytosine to uracil reported in the prior art are available, including one or several of hydrazonium salt, bisulfite, and hydrosulfite (sodium metabisulfite, potassium bisulfite, cesium bisulfite, and ammonium bisulfite). Another example is DNA polymerase, dNTPs, Mg²⁺ ions, buffer, *etc.,* which are common in gene amplification.

In some embodiments, the reagent or the kit further includes a detection reagent of a reference gene.

In some embodiments, the reference gene is β-actin.

In some embodiments, the detection reagent of the reference gene refers to a primer and a probe specified at the reference gene.

In some embodiments, the detection reagent of the reference gene is a primer pair shown as SEQ ID NO: 13 and SEQ ID NO: 14, and a probe is shown as SEQ ID NO: 15.

The present disclosure provides a use of the combined methylation detection reagent of the HOXB4 and SRCIN1 genes in preparation of a lung cancer detection reagent or kit.

The present disclosure provides a primer. The primer is selected from a nucleotide sequence having an identity of at least 85% or at least 90% or at least 91% or at least 92% or at least 93% or at least 94% or at least 95% or at least 96% or at least 97% or at least 98% or at least 99% or 100% to nucleotide sequences shown as SEQ ID NO: 1, SEQ ID NO: 2, SEQ ID NO: 4, SEQ ID NO: 5, SEQ ID NO: 16, SEQ ID NO: 17, SEQ ID NO: 19, SEQ ID NO: 20 or SEQ ID NO: 22, or at least any of complementary sequences of the sequences.

The present disclosure provides a primer. The primer is selected from a nucleotide sequence having an identity of at least 85% or at least 90% or at least 91% or at least 92% or at least 93% or at least 94% or at least 95% or at least 96% or at least 97% or at least 98% or at least 99% or 100% to nucleotide sequences shown as SEQ ID NO: 1, SEQ ID NO: 2, SEQ ID NO: 4 or SEQ ID NO: 5, or at least any of complementary sequences of the sequences.

In some embodiments, the primer is selected from at least one primer pair shown as SEQ ID NO: 1 and SEQ ID NO: 2, or SEQ ID NO: 4 and SEQ ID NO: 5.

In some embodiments, the primer is selected from primer pairs shown as SEQ ID NO: 1 and SEQ ID NO: 2, or SEQ ID NO: 4 and SEQ ID NO: 5.

The primer is used for amplifying nucleic acid fragments. It is well known in the art that, the successful design of the primer is of crucial importance on the PCR. Relative to general PCR, the design impact of the primer is more crucial in methylation detection. The reason is as follows: "C" in a DNA strand is promoted to transform into "U" due to a methyl sulfuration reaction, and then GC content is decreased. Thus, long continuous "T" appears in the sequence after the PCR; DNA strand breakage is easily caused, and it is difficult to select a stable primer having an appropriate Tm value. Further, to distinguish sulfidized DNA and DNA that is not sulfidized or completely treated, the primer needs to have sufficient "C". Thus, the difficulty of selecting the stable primer is increased. Therefore, in DNA methylation detection, the selection of specified amplification fragments of the primer, such as lengths and locations of the amplification fragments, and selection of the primer have effects on detection sensitivity and specificity. Through experiments, the inventor finds that different target amplification fragments and primers have different detection effects. Many times, it is discovered that some genes or nucleic acid fragments have expression differences in tumors and non-tumors. However, there is still a very long distance in transforming the genes or nucleic acid fragments into a tumor marker for clinical application. The most important reason is as follows: due to the limitation of the detection reagent, detection sensitivity and specificity of the potential tumor marker are difficult to meet detection requirements, or the detection method is complex in operation and high in cost and is difficult to be clinically applied on a large scale.

In another aspect, the present disclosure further provides a nucleic acid probe. The nucleic acid probe is selected from a nucleotide sequence having an identity of at least 85% or at least 90% or at least 91% or at least 92% or at least 93% or at least 94% or at least 95% or at least 96% or at least 97% or at least 98% or at least 99% or 100% to sequences shown as SEQ ID NO: 3, SEQ ID NO: 6, SEQ ID NO: 18 and SEQ ID NO: 21, or at least any of complementary sequences of the sequences.

As a preferred embodiment of the present disclosure, the nucleic acid probe is selected from the sequences shown as SEQ ID NO: 3 and SEQ ID NO: 6.

In some embodiments, the kit provided by the present disclosure includes a first container including a primer pair used for amplification, and a second container including a probe.

In some embodiments, the kit further includes the instruction.

In some embodiments, the kit further includes a nucleic acid extraction reagent.

In some embodiments, the kit further includes a sampling device.

The present disclosure further provides a use of the above methylation detection reagent, the kit, the primer, and the probe, in preparation of a methylation detection reagent or kit, or in preparation of a lung cancer detection reagent or kit.

The present disclosure further provides a use of the above methylation detection reagent, the kit, the primer, and the probe in methylation detection or use in detection of the lung cancer.

The present disclosure further provides a lung cancer detection system. The system includes the following components:
(1) a combined methylation detection component of HOXB4 and SRCIN1 genes;
(2) a data processing component; and
(3) a result output component.

In some embodiments, the methylation detection component includes a methylation detection instrument.

In some embodiments, the methylation detection component further includes the methylation detection instrument, the kit, the primer, and the probe.

In some embodiments, the methylation detection instrument includes one or more of a fluorescent quantitative PCR instrument, a PCR instrument, and a sequencer.

In some embodiments, the data processing component includes a data processing machine.

The data processing machine includes any device or instrument or apparatus that can be used by those skilled in the art and that can conduct data processing.

In some embodiments, the data processing machine includes one or more calculators and computers.

The computers are loaded with any software or program that can be used by those skilled in the art and that can conduct data processing or statistical analysis.

In some embodiments, the computers include a computer loaded with one or more pieces of software in SPSS, SAS, and Excel.

In some embodiments, the result output component includes a result output unit.

The output unit includes any device or instrument or apparatus that can display data processing results into readable contents.

In some embodiments, the methylation detection component further includes the multi-gene combined methylation detection reagent.

In some embodiments, the result output unit includes one or more screens and paper reports.

In some embodiments, the data processor is configured to: a. receive test data of sample to be detected and normal control sample; b. store the test data of the sample to be detected and the normal control sample; c. compare the test data of the sample to be detected and the normal control sample of the same type; d. respond to the probability or possibility of the subject suffering from the lung cancer according to comparison results.

In some embodiments, the result output component is used for outputting the probability or possibility of the subject suffering from the lung cancer.

In some embodiments, a criterion of the data processing component is as follows: lung cancer specimens and normal specimens are determined according to a boundary value.

In some embodiments, combined detection of the HOXB4 and SRCIN1 in the present disclosure can be realized in a multiple PCR mode.

In some embodiments, a methylation level of the specimens is determined according to a Cp value and/or a ΔCp value of target genes, that is, the HOXB4 and SRCIN1 (ΔCp value = Cp_{target gene} - Cp _{reference gene}).

In some embodiments, the tissue specimens are determined as the lung cancer specimens as long as the ΔCp value of one gene in the tissue specimens is less than the boundary value of the ΔCp value; and the tissue specimens are determined as the normal specimens only when the ΔCp values of the two genes in the tissue specimens are simultaneously more than or equal to the boundary value of the ΔCp value.

In some embodiments, a boundary value of the Cp value in the specimens ranges from 35 to 39; and the boundary value of the ΔCp value ranges from 4 to 12.

In some embodiments, during the combined detection of the HOXB4 and SRCIN1, in the tissue specimens, the boundary value of the ΔCp value of the HOXB4 is 5.4, and the boundary value of the ΔCp value of the SRCIN1 is 6.5.

In some embodiments, during the combined detection of the HOXB4 and SRCIN1, in sputum specimens, threshold Cp values of the HOXB4 and SRCIN1 are respectively 36.9 and 37.0. When one of the individual gene detection results is less than the above threshold, the specimens can be identified as positive (that is, the lung cancer specimens); and if 2 items of the detection results are more than or equal to the corresponding threshold values, the specimens can be identified as negative (that is, the normal specimens).

In some embodiments, during multiple PCR detection of the HOXB4 and SRCIN1, the boundary value of the Cp value in the sputum specimens is 36.7, while the boundary value of the Cp value is 37.2 and the boundary value of the ΔCp value is 9 in lavage fluid specimens.

In some embodiments, during multiple PCR detection of the HOXB4 and SRCIN1, the specimens are determined as the lung cancer specimens when the Cp value of the sputum specimens is less than the boundary value of the Cp value; and the specimens are determined as the normal specimens when the Cp value of the sputum specimens is more than or equal to the boundary value of the Cp value.

In some embodiments, during multiple PCR detection of the HOXB4 and SRCIN1, the specimens are determined as the lung cancer specimens when any numerical value of the Cp value and the ΔCp value of the lavage fluid specimens is less than the boundary value of the Cp value and the ΔCp value; and the specimens are determined as the normal specimens when the Cp value and the ΔCp value of the lavage fluid specimens are more than or equal to the boundary value of the Cp value and the ΔCp value.

In some embodiments, the tumor is the lung cancer.

In some embodiments, the tumors are the small cell lung cancer and the non-small cell lung cancer.

In some embodiments, the non-small cell lung cancer is selected from squamous-cell carcinoma or adenomatous carcinoma.

In some embodiments, types of the specified sample to be detected or sample is selected from at least one of the pulmonary alveoli lavage fluid, tissues, hydrothorax, sputum, blood, serum, plasma, urine, prostatic fluid, or excrements.

In some embodiments, the samples in the present disclosure are selected from at least one of the pulmonary alveoli lavage fluid, tissues, or sputum.

In some embodiments, the samples in the present disclosure are selected from at least one of the pulmonary alveoli lavage fluid or sputum.

The present disclosure further provides a lung cancer diagnosis method. The method includes the following steps:
(1) detecting methylation levels of HOXB4 and SRCIN1 genes in a sample to be detected derived from a subject;
(2) comparing methylation levels of HOXB4 and SRCIN1 genes of the sample to be detected and normal control sample; and
(3) diagnosing the lung cancer based on a deviation of the methylation levels of the sample to be detected and normal control sample.

In some embodiments, the present disclosure provides a lung cancer diagnosis method. The method includes the following steps: (1) detecting methylation levels of HOXB4 and SRCIN1 genes in a sample to be detected derived from a subject, wherein the detection includes contact between the sample to be detected of the subject and a detection reagent for detecting the methylation levels of the HOXB4 and SRCIN1 genes; (2) comparing the methylation levels of the HOXB4 and SRCIN1 genes of the sample to be detected and normal control sample; and (3) diagnosing the lung cancer based on a deviation of the methylation levels of the sample to be detected and the normal control sample.

In some embodiments, the present disclosure provides a lung cancer diagnosis method. The method includes the following steps: adding a methylation detection reagent of genes into the sample to be detected derived from a subject, and detecting methylation levels of HOXB4 and SRCIN1 genes in the sample to be detected; comparing the methylation levels of the HOXB4 and SRCIN1 genes of the sample to be detected and normal control sample; and diagnosing the lung cancer based on the deviation of the methylation levels of the sample to be detected and the normal control sample.

In some embodiments, the deviation in step (3) refers to the deviation of the methylation level of any of the 2 genes, that is, the HOXB4 and SRCIN1.

In some embodiments, in step (1), the detection includes contact between the sample to be detected of the subject and the detection reagent for detecting the methylation levels of the HOXB4 and SRCIN1 genes.

In some embodiments, the methylation levels of the HOXB4 and SRCIN1 genes are detected by using quantitative methylation-specific PCR (qMSP).

In some embodiments, methylation results of the sample to be detected and the normal sample are compared through the results; and when the results of the sample to be detected and the normal sample have significant differences or extremely significant differences, it is determined from the results that, the sample to be detected have high disease risk.

The diagnosis method in the present disclosure can be used before and after lung cancer treatment or be combined with lung cancer treatment. When used after the treatment, the diagnosis method includes: evaluating the success of the treatment, or monitoring alleviation, reoccurrence, and/or progress (including metastasis) of the lung cancer after the treatment.

In one aspect, a lung cancer treatment method is further provided. The method includes the following steps:
(1) detecting methylation levels of HOXB4 and SRCIN1 genes in a sample to be detected derived from a subject;
(2) comparing methylation levels of HOXB4 and SRCIN1 genes of the sample to be detected and normal control sample;
(3) diagnosing the lung cancer based on a deviation of the methylation levels of the sample to be detected and normal control sample; and
(4) applying an anti-lung-cancer drug to the subject diagnosed with the lung cancer.

Another aspect of the present disclosure provides a lung cancer treatment method. The method includes: applying operations, chemotherapy, radiotherapy, chemoradiotherapy, immunotherapy, oncolytic virus therapy, or any other treatment method for other types of lung cancers available in the art and a combination of these treatment methods to patients diagnosed with the lung cancer by the above diagnosis method.

Through research, it is found in the present disclosure that, in some specific embodiments, lung cancer samples can be well distinguished from the samples by detecting a combination of the HOXB4 and SRCIN1 genes. The detection sensitivity and specificity on lung cancer are extremely high.

### BRIEF DESCRIPTION OF THE DRAWINGS

Fig. 1 is the ROC curves of different marker combinations for detecting tissue samples;
Fig. 2 is the ROC curves of different marker combinations for detecting sputum samples; and
Fig. 3 is an amplification curve of HOXB4 and SRCIN1 combined detection for detecting lavage fluid specimens.

### DETAILED DESCRIPTION

Technical solutions in the present disclosure will be further described below by virtue of specific examples. Specific examples do not represent a limitation to the protection scope of the present disclosure. Some nonessential modifications and adjustments made by other persons according to the concept of the present disclosure still belong to the protection scope of the present disclosure.

"Primer" or "probe" in the present disclosure refers to an oligonucleotide, including a region complementary to a sequence of at least 6 continuous nucleotides of a target molecule (such as a target nucleic acid fragment). In some embodiments, at least one part of the sequence of the primer or the probe is not complementary to an amplified sequence. In some embodiments, the primer or the probe includes a region complementary to a sequence of at least 9, at least 10, at least 11, at least 12, at least 13, at least 14, at least 15, at least 16, at least 17, at least 18, at least 19 or at least 20 continuous nucleotides of the target molecule. When the primer or the probe includes a region "complementary to at least *x* continuous nucleotides of the target molecule", the primer or the probe is at least 95% complementary to at least *x* continuous or discontinuous block nucleotides of the target molecule. In some embodiments, the primer or the probe is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% complementary to the target molecule.

In the present disclosure, "normal" samples refer to samples of the same type isolated from known individuals having no cancers or tumors.

Methylation detection samples in the present disclosure include but not limited to DNA, or RNA, or mRNA-containing DNA and RNA samples or DNA-RNA hybrids, wherein the DNA or RNA may be single-stranded or double-stranded.

In the present disclosure, the "subjects" are mammals, such as persons.

In the present disclosure, the "methylation level" is the same as the "methylation degree", and can be generally represented as a percentage of methylated cytosine, wherein the percentage of methylated cytosine is obtained by dividing a quantity of the methylated cytosine by the sum of the quantity of the methylated cytosine and a quantity of non-methylated cytosine. Further, at present, the methylation level is presented by generally using a method for dividing the number of methylated target genes by the number of reference genes. Moreover, the methylation level is presented by other methylation level representation methods in the prior art.

"Samples" in the present disclosure are the same as "specimens".

The term "and/or" used in the present disclosure refers to and covers any of one or more associated listed items and any possible combination. When used in a list of two or more items, the term "and/or" represents that any of the listed items can be used alone, or any combination of two or more listed items can be used. For example, if a composition, a combination, or a structure is described to comprise (or include) components of A, B, C, and/or D, the composition may separately include the A, separately include the B, separately include the C and separately include the D; and may include a combination of A and B, a combination of A and C, a combination of A and D, a combination of B and C, a combination of B and D, a combination of C and D, a combination of A, B and C, a combination of A, B and D, a combination of A, C and D, a combination of B, C and D, or a combination of A, B, C and D.

### Example 1

Hundreds of genes are screened by the inventor. The genes are screened from tissue samples, a gene β-actin serves as a reference gene, combined detection results of every two of genes such as HOXB4, SRCIN1, PCDHGA12, and HOXD8 are compared, and detection primers and probes of each gene are as follows:
The detection primers and probe of HOXB4 are:
   SEQ ID NO: 1 HOXB4-F1 primer F: TTCGTCGTTTTCGTTATCATTC
   SEQ ID NO: 2 HOXB4-R1 primer R: TACTAACCGCCTCGCTAC
   SEQ ID NO: 3 HOXB4-P1 probe P: FAM-CGGGTTTTTGCGTCGTTATTCGTC-BQ1
The detection primers and probe of SRCIN1 are:
   SEQ ID NO: 4 SRCIN1 primer F: TCGTGTGTCGTCGTTCAGAC
   SEQ ID NO: 5 SRCIN1 primer R: GAAATACCCGCGAAAATACTG
   SEQ ID NO: 6 SRCIN1 probe P: FAM-AGTTTTACGTTGGAGAAGCGTCGG-BQ1
The detection primers and probe of PCDHGA12 are:
   SEQ ID NO: 7 PCDHGA12 primer F: TTGGTTTTTACGGTTTTCGAC
   SEQ ID NO: 8 PCDHGA12 primer R: AAATTCTCCGAAACGCTCG
   SEQ ID NO: 9 PCDHGA12 probe P: FAM-ATTCGGTGCGTATAGGTATCGCGC-BQ1
The detection primers and probe of HOXD8 are:
   SEQ ID NO: 10 HOXD8 primer F: TTAGTTTCGGCGCGTAGC
   SEQ ID NO: 11 HOXD8 primer R: CCTAAAACCGACGCGATCTA
   SEQ ID NO: 12 HOXD8 probe P: FAM-AARACTTACGATCGTCTACCCTCCG-BQ1
The detection primers and probe of β-actin are:
   SEQ ID NO: 13 β-actin primer F: GGAGGTTTAGTAAGTTTTTTGGATT
   SEQ ID NO: 14 β-actin primer R: CAATAAAACCTACTCCTCCCTTA
   SEQ ID NO: 15 β-actin probe P: FAM-TTGTGTGTTGGGTGGTGGTT-BQ1

### Experimental procedures:

### 1. Extraction of DNA

Specimens of diagnosed lung cancer patients and specimens of non-lung-cancer patients are collected, and the specimens respectively include paraffin-embedded tissue specimens, sputum specimens, and lavage fluid specimens. After sample pretreatment and isolation of cells, DNA extraction is conducted according to the instruction of HiPure FFPE DNA Kit (D3126-03) in Magen Company.

### 2. DNA modification

Bisulfite modification is conducted according to the instruction of EZ DNA Methylation ^{™} KIT (D5002) in ZYMO RESEARCH Biotechnology Company.

### 3. Amplification and detection

**Table 1 Solution preparation system**

| | HOXB4 | SRCIN1 | PCDHGA12 | HOXD8 | β-actin |
|---|---|---|---|---|---|
| **Reaction components** | Addition (µl) | Addition (µl) | Addition (µl) | Addition (µl) | Addition (µl) |
| Forward primer (100 µM) | 0.125 | 0.125 | 0.125 | 0.05 | 0.125 |
| Reverse primer (100 µM) | 0.125 | 0.125 | 0.125 | 0.125 | 0.125 |
| Probe (100 µM) | 0.05 | 0.05 | 0.05 | 0.05 | 0.05 |
| Magnesium ion (25 mM) | 6 | 6 | 6 | 6 | 6 |
| dNTPs (10 mM) | 1 | 1 | 1 | 1 | 1 |
| Taq polymerase (5 unit/µl) | 0.5 | 0.5 | 0.5 | 0.5 | 0.5 |
| 5× buffer | 6 | 6 | 6 | 6 | 6 |
| Sterile water | 11.2 | 11.2 | 11.2 | 11.275 | 11.2 |
| Template DNA | 5 | 5 | 5 | 5 | 5 |
| Total volume | 30 | 30 | 30 | 30 | 30 |

**Amplification system:** amplification systems of the various detection genes are shown in Table 2 and Table 3.

**Table 2 Amplification systems of HOXB4, SRCIN1m and β-actin**

| Steps | Temperature and time | Number of cycles |
|---|---|---|
| Pre-denaturation | 95°C 5 minutes | 1 |
| Amplification | 95°C 15 seconds | 48 |
| | 58°C 30 seconds | |
| | 72°C 30 seconds | |
| Cooling | 40°C 30 seconds | 1 |

**Table 3 Amplification systems of PCDHGA12 and HOXD8**

| Steps | Temperature and time | Number of cycles |
|---|---|---|
| Pre-denaturation | 95°C 5 minutes | 1 |
| Amplification 1 | 95°C 20 seconds | 10 |
| | 60°C 30 seconds | |
| | 70°C 30 seconds | |
| Amplification 2 | 95°C 20 seconds | 45 |
| | 55°C 60 seconds | |
| | 72°C 30 seconds | |
| Cooling | 40°C 30 seconds | 1 |

### 4. Detection results

Sample information: there are a total of 169 lung tissue samples, including 91 normal tissue samples and 78 cancer tissue samples. The 78 cancer tissue samples include 27 squamous carcinoma samples, 38 adenomatous carcinoma samples, 3 small cell cancer samples, 4 large cell cancer samples, 1 compound cancer sample, and 5 lung cancer samples that are not clearly classified. There are 77 pairs of cancer and cancer-adjacent control samples.

ACTB serves as a reference gene; methylation levels of specimens are determined according to ΔCp values of target genes, that is, the HOXB4 and SRCIN1 (ΔCp value = Cp_{target gene} - Cp_{ACTB}); and thresholds of the HOXB4 and SRCIN1 are respectively as follows: ΔCp value = 5.4, and ΔCp value = 6.5. When one item in the detection results is less than the above threshold, the specimens can be identified as positive; and if 2 items in the detection results are more than or equal to the corresponding thresholds, the specimens can be identified as negative.

A threshold of the PCDHGA12 is Cp value = 25.9, and a threshold of the HOXD8 is Cp value = 27.4. When the detection result of each marker is more than or equal to the corresponding threshold, the specimens can be identified as negative; and when the detection result of each marker is less than the corresponding threshold, the specimens can be identified as positive.

The ROC curves of a combination of HOXB4 and SRCIN1, a combination of HOXB4 and PCDHGA12, and a combination of HOXB4 and HOXD8 for testing all the tissue samples are shown in Fig. 1. Statistical results of each gene tested in the tissues are shown in Table 3.

**Table 3 Detection results in tissues**

| **Analytical groups** | **Indicator** | HOXB4 | SRCIN1 | PCDHGA12 | HOXD8 |
|---|---|---|---|---|---|
| Comparison of normal groups and total cancer groups | Specificity | 100% | 97.8% | 97.8% | 97.8% |
| | Sensitivity | 73.1% | 71.8% | 50.0% | 53.8% |
| Comparison of normal groups and squamous carcinoma groups | Specificity | 100% | 97.8% | 97.8% | 97.8% |
| | Sensitivity | 74.1% | 51.9% | 44.4% | 81.5% |
| Comparison of normal groups and adenomatous carcinoma groups | Specificity | 100% | 97.8% | 97.8% | 97.8% |
| | Sensitivity | 78.9% | 89.5% | 50.0% | 50% |
| Comparison of normal groups and small cell cancer groups | Specificity | 100% | 97.8% | 97.8% | 97.8% |
| | Sensitivity | 0% | 33.3% | 66.7% | 33.3% |
| Comparison of normal groups and large cell cancer groups | Specificity | 100% | 97.8% | 97.8% | 97.8% |
| | Sensitivity | 50.0% | 75.0% | 50.0% | 0% |

**Table 3 (continued)**

| **Analytical groups** | **Indicator** | Combination of HOXB4 and SRCIN1 | Combination of HOXB4 and PCDHGA12 | Combination of HOXB4 and HOXD8 |
|---|---|---|---|---|
| Comparison of normal groups and total cancer groups | Specificity | 97.8% | 97.8% | 97.8% |
| | Sensitivity | 89.7% | 75.6% | 78.2% |
| Comparison of normal groups and squamous carcinoma groups | Specificity | 97.8% | 97.8% | 97.8% |
| | Sensitivity | 81.5% | 74.1% | 81.5% |
| Comparison of normal groups and adenomatous carcinoma groups | Specificity | 97.8% | 97.8% | 97.8% |
| | Sensitivity | 100% | 78.9% | 81.6% |
| Comparison of normal groups and small cell cancer groups | Specificity | 97.8% | 97.8% | 97.8% |
| | Sensitivity | 33.3% | 66.7% | 33.3% |
| Comparison of normal groups and large cell cancer groups | Specificity | 97.8% | 97.8% | 97.8% |
| | Sensitivity | 75.0% | 50.0% | 50.0% |

It can be seen from the above results that, through comparison of the normal groups and total cancer groups, the detection of the combination of HOXB4 and SRCIN1 in tissue samples has a specificity of 97.8% and the sensitivity of 89.7%; and compared with the detection of two another combinations, the detection of the combination of HOXB4 and SRCIN1 has higher sensitivity in case of the consistent specificity. In addition, compared with separate detection of the HOXB4 and SRCIN1, not obviously, the detection of the combination of HOXB4 and SRCIN1 significantly improves the sensitivity.

According to the above results, the HOXB4 and SRCIN1 still have higher sensitivity in the tissue samples under high specificity. Particularly, through the combined detection, the sensitivity is greatly improved under the condition that the specificity is basically not affected. Sputum serving as a non-invasive detection sample has significance in lung cancer diagnosis. Therefore, the 2 markers, namely the HOXB4 and SRCIN1, are detected in the sputum by the inventor.

### Example 2: Detection of HOXB4 and SRCIN1 genes in sputum

Sample information: there are a total of 107 tested sputum samples, including 51 normal control group samples and 56 cancer group samples. The 56 cancer group samples include 20 squamous carcinoma samples, 8 small cell cancer samples, 20 adenomatous carcinoma samples, 1 large cell cancer sample, 1 giant cell carcinoma sample, and 6 lung cancer samples that are not clearly classified.

Test procedures:
a. Sputum specimens of diagnosed lung cancer patients and non-lung-cancer patients are collected; the specimens are diluted with NaOH and are centrifugally precipitated to isolate cells; the specimens are washed with PBS twice; and then DNA is extracted by using a DNA extraction kit (HiPure FFPE DNA Kit D3126-03) in Magen Company.
b. The DNA is subjected to bisulfite modification by using a DNA transformation kit (EZ DNA Methylation Kit, D5002) in ZYMO RESEARCH Biotechnology Company.
c. Primer and probe sequences, a solution preparation system and an amplification system for each gene marker are the same as those in Example 1.
d. Methylation levels of the specimens are determined according to Cp values of target genes, namely, the HOXB4 and SRCIN1; and threshold Cp values of the HOXB4 and SRCIN1 are respectively 36.9 and 37.0. When one item in the detection results of a single gene is less than the above threshold, the specimens can be identified as positive; and if 2 items in the detection results are more than or equal to the corresponding thresholds, the specimens can be identified as negative.
PCDHGA12 and HOXD8: the threshold of the PCDHGA12 is Cp value = 23.48; the threshold of the HOXD8 is Cp value = 26.4; when the detection result of each marker is more than or equal to the corresponding threshold, the specimens can be identified as negative; and when the detection result of each marker is less than the corresponding threshold, the specimens can be identified as positive.
e. The detection results are as follows:

**Table 4 Detection results in sputum**

| **Analytical groups** | **Indicator** | **HOXB4** | **SRCIN1** | **PCDHGA12** | **HOXD8** |
|---|---|---|---|---|---|
| | Specificity | 96.1% | 96.1% | 96.1% | 96.1% |
| Comparison of normal groups and total cancer groups | Sensitivity | 64.3% | 48.2% | 16.1% | 23.2% |
| Comparison of normal groups and squamous carcinoma groups | Specificity | 96.1% | 96.1% | 96.1% | 96.1% |
| | Sensitivity | 80.0% | 35.0% | 25.0% | 50.0% |
| Comparison of normal groups and adenomatous carcinoma groups | Specificity | 96.1% | 96.1% | 96.1% | 96.1% |
| | Sensitivity | 50.0% | 40.0% | 10.0% | 5.0% |
| Comparison of normal groups and small cell cancer groups | Specificity | 96.1% | 96.1% | 96.1% | 96.1% |
| | Sensitivity | 50.0% | 100% | 12.5% | 12.5% |

**Table 4 (continued)**

| **Analytical groups** | **Indicator** | **Combination of HOXB4 and SRCIN1** | **Combination of HOXB4 and PCDHGA12** | **Combination of HOXB4 and HOXD8** |
|---|---|---|---|---|
| Comparison of normal groups and total cancer groups | Specificity | 92.2% | 92.2% | 94.1% |
| | Sensitivity | 76.8% | 64.3% | 66.1% |
| Comparison of normal groups and total squamous carcinoma groups | Specificity | 92.2% | 92.2% | 94.1% |
| | Sensitivity | 85.0% | 80.0% | 85.0% |
| Comparison of normal groups and total adenomatous carcinoma groups | Specificity | 92.2% | 92.2% | 94.1% |
| | Sensitivity | 55.0% | 50.0% | 50.0% |
| Comparison of normal groups and total small cell cancer groups | Specificity | 92.2% | 92.2% | 94.1% |
| | Sensitivity | 100% | 50.0% | 50.0% |

The ROC curves of the HOXB4 and SRCIN1 for testing the sputum specimens are shown in Fig. 2, and statistical results are shown in Table 4. It can be seen from the above results that, through the comparison of normal groups and total cancer groups, during the detection of the combination of HOXB4 and SRCIN1 in the sputum samples, the sensitivity on the lung cancer is improved to 76.8%; and through the comparison of normal groups and total small cell cancer groups, the sensitivity can be up to 100%. Relative to a single gene marker, the detection rate of the HOXB4 is 64.3%, and the detection rate of the SRCIN1 is 48.2%. During the combined detection of the two genes, the sensitivity on the lung cancer is improved to 76.8%; and the two genes have synergism.

### Example 3: Optimization of multiplex PCR systems of HOXB4 and SRCIN1 genes

The above results show that the combined detection of the HOXB4 and SRCIN1 genes can significantly improve the detection rate of lung cancer. The PCR system is optimized by the inventor in a multiple PCR mode, thereby simplifying detection procedures and conducting validation on the basis of the samples in Example 2. Detection primers and probes of the various genes are as follows:
Sequences of detection primers and probes of the HOXB4, SRCIN1, and β-actin are the same as those in Example 1.
a. Sputum sample treatment is the same as in Example 2.
b. The solution preparation system is as follows:

**Table 5 Solution preparation system**

| **Reaction component** | Addition (µl) |
|---|---|
| HOXB4-F1(100 µM) | 0.125 |
| HOXB4-R1(100 µM) | 0.125 |
| HOXB4-P1(100 µM) | 0.05 |
| SRCIN1-F1(100 µM) | 0.125 |
| SRCIN1-R1(100 µM) | 0.125 |
| SRCIN1-P1(100 µM) | 0.05 |
| β-actin-F1(100 µM) | 0.125 |
| β-actin-R1(100 µM) | 0.125 |
| β-actin-P2(100 µM) | 0.05 |
| Magnesium ion (25 mM) | 6 |
| dNTPs (10 mM) | 1 |
| Taq polymerase (5 unit/µl) | 0.5 |
| 5× buffer | 6 |
| Sterile water | 10.6 |
| Template DNA | 5 |
| Total volume | 30 |

c. The amplification system is the same as the amplification system in Table 2 of Example 1.
d. Methylation levels of the specimens are determined according to Cp values of multiple PCR of target genes, namely, the HOXB4 and SRCIN1; and the threshold Cp value of the multiple PCR detection of the HOXB4 and SRCIN1 is 36.7. When one item in the detection results of a single gene is less than the above threshold, the specimens can be identified as positive; and if the detection results are more than or equal to the threshold, the specimens can be identified as negative.
e. Detection results are shown in Table 6:

**Table 6 Detection results**

| **Analytical groups** | **Indicator** | Combined detection of HOXB4 and SRCIN1 |
|---|---|---|
| Comparison of normal groups and total cancer groups | Specificity | 94.1% |
| | Sensitivity | 78.6% |
| Comparison of normal groups and total squamous carcinoma groups | Specificity | 94.1% |
| | Sensitivity | 85.0% |
| Comparison of normal groups and total adenomatous carcinoma groups | Specificity | 94.1% |
| | Sensitivity | 60.0% |
| Comparison of normal groups and total small cell cancer groups | Specificity | 94.1% |
| | Sensitivity | 100% |

The results show that, the detection results of the multiple PCR system of the HOXB4 and SRCIN1 are basically consistent with the detection results in Example 2. Thus, it is indicated that, the detection results of the multiple PCR system can serve as the determined results of the combination of the HOXB4 and SRCIN1 genes for detecting lung cancer.

### Example 4: Detection of HOXB4 and SRCIN1 genes in lavage fluid

Sample information: there are a total of 387 tested pulmonary alveoli lavage fluid samples, wherein the samples include 303 normal control group samples and 84 cancer group samples. The 84 cancer group samples include 21 squamous carcinoma samples, 40 adenomatous carcinoma samples, 10 small cell cancer samples and 13 lung cancer samples that are not clearly classified.

Sequences of detection primers and probes of the HOXB4, SRCIN1 and β-actin are the same as those in Example 1.

### Test procedures:

a. Pulmonary alveoli lavage fluid specimens of diagnosed lung cancer patients and non-lung-cancer patients are collected; the specimens are centrifuged to isolate cells, and then DNA is extracted by using a DNA extraction kit (HiPure FFPE DNA Kit D3126-03) in Magen Company.
b. The DNA is subjected to bisulfite modification by using a DNA transformation kit (EZ DNA Methylation Kit, D5002) in ZYMO RESEARCH Biotechnology Company.
c. A solution preparation system and an amplification system are the same as those in example 3.
d. Detection results are as follows:
ACTB serves as a reference gene; methylation levels of the specimens are determined according to a Cp value and a ΔCp value of target genes, that is, the HOXB4 and SRCIN1 (ΔCp value = Cp_{target gene} - Cp_{ACTB}); and thresholds of the HOXB4 and SRCIN1 are as follows: Cp value = 37.2, and ΔCp value = 9. When one item in the detection results is less than the above threshold, the specimens can be identified as positive; and if 2 items in the detection results are more than or equal to the thresholds, the specimens can be identified as negative. The detection results of the 387 pulmonary alveoli lavage fluid specimens are as follows:

**Table 7 Detection results**

| **Analytical groups** | **Indicator** | Combination of HOXB4 and SRCIN1 |
|---|---|---|
| Comparison of normal groups and total cancer groups | Specificity | 96.0% |
| | Sensitivity | 77.4% |
| Comparison of normal groups and total squamous carcinoma groups | Specificity | 96.0% |
| | Sensitivity | 71.4% |
| Comparison of normal groups and total adenomatous carcinoma groups | Specificity | 96.0% |
| | Sensitivity | 75.0% |
| Comparison of normal groups and total small cell cancer groups | Specificity | 96.0% |
| | Sensitivity | 90.0% |

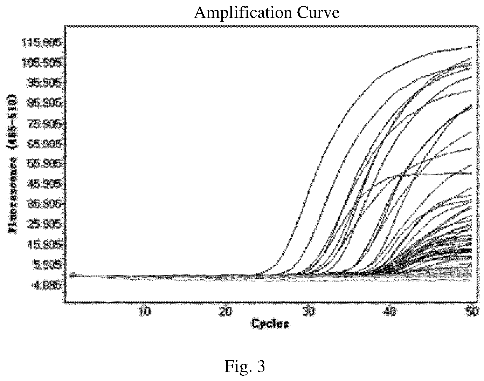

An amplification curve of the combination of HOXB4 and SRCIN1 for testing all the lavage fluid specimens is shown in Fig. 3, and statistical results are shown in Table 7. It can be seen from the above results that, the detection of the combination of HOXB4 and SRCIN1 reaches the sensitivity of 77.4% at high specificity of 96.0%; and through comparative analysis according to subtypes of lung cancer, a detection rate of the detection of the combination of HOXB4 and SRCIN1 is up to 71.4% in the squamous carcinoma group. Particularly for the detection effect of adenomatous carcinoma, the sensitivity of the detection of the combination of HOXB4 and SRCIN1 is up to 75.0%. This breakthrough has significance in the detection of adenomatous carcinoma. Since adenomatous carcinoma is generally peripheral, due to a tree-like physiological structure of the bronchus, the pulmonary alveoli lavage fluid is difficult to contact with pulmonary alveoli or cancer tissues deep into the lung.

### Example 5 Detection results of different marker combinations in sputum samples

The inventor simultaneously compares the detection conditions of different marker combinations in the sputum samples. Compared groups are as follows:
Group 1: HOXB4+SRCIN1
Group 2: HOXB4+PCDHGA12
Group 3: SRCIN1+PCDHGA12
Group 4: HOXB4+HOXD8
Group 5: SRCIN1+HOXD8
Group 6: SRCIN1+PCDHGA12+HOXD8
Group 7: SRCIN1+HOXB4+HOXD8

Specific experimental conditions and operations are the same as those in Example 2.

The different marker combinations are detected in 107 sputum samples. The sputum samples include 51 normal control group samples and 56 cancer group samples. The detection results of each group are shown in Table 8.

**Table 8 (Normal group vs. total cancer groups)**

| | Combination 1 | Combination 2 | Combination 3 | Combination 4 | Combination 5 | Combination 6 | Combination 7 |
|---|---|---|---|---|---|---|---|
| Sensitivity | 76.8% | 64.3% | 46.4% | 66.1% | 53.6% | 53.6% | 76.8% |
| Specificity | 92.2% | 92.2% | 92.2% | 94.1% | 94.1% | 90.2% | 92.2% |
| AUC | 0.870 | 0.810 | 0.848 | 0.833 | 0.883 | 0.832 | 0.892 |

The results in Table 8 show that the different marker combinations have significant effects on the lung cancer detection rate of the sputum samples. Analytical results of Combination 1, Combination 2, Combination 3, Combination 4, and Combination 5 show that the detection results of combinations of every two different markers have great differences; in Combination 1, the detection of the combination of the HOXB4 and SRCIN1 has the specificity of 92.2% and the sensitivity of 76.8%; and comprehensive detection performance of Combination 1 is far higher than that of any of the rest 4 combinations. Through analysis of Combination 3, Combination 5, and Combination 6, or Combination 1 and Combination 7, the results show that the detection rate is not necessarily improved when an extra marker is increased, on the contrary, the specificity is possibly decreased. The results show that not any gene marker combination can achieve comprehensively excellent effects in the 3 indicators, such as the sensitivity, specificity and AUC, just like Combination 1 in the present disclosure. Not obviously, the specificity and the sensitivity are not correspondingly improved by increasing the quantity of target gene markers for conducting combined detection (such as Combination 6 and Combination 7).

### Example 6 Selection of primer and probe combinations

Primers and probes have significant effects on the detection effects of tumor markers. Multiple pairs of primers and corresponding probes thereof are designed by the inventor during the research to find primers and probes that can improve the detection sensitivity and specificity as much as possible. Thus, the detection reagent in the present invention can be actually applied to clinical detection.

Different primer and probe combinations are detected in 40 sputum samples. The sputum samples include 15 normal control group samples and 25 cancer group control samples.

**Table 9 Primers and probes**

| Name | Sequence No. | Sequence | Effects |
|---|---|---|---|
| HOXB4-F1 | SEQ ID NO: 1 | TTCGTCGTTTTCGTTATCATTC | HOXB4 forward primer |
| HOXB4-R1 | SEQ ID NO: 2 | TACTAACCGCCTCGCTAC | HOXB4 reverse primer |
| HOXB4-P1 | SEQ ID NO: 3 | FAM-CGGGTTTTTGCGTCGTTATTCGTC-BQ1 | HOXB4 detection probe |
| HOXB4-F2 | SEQ ID NO: 16 | ATTCGTTCGGGTATTACGTC | HOXB4 forward primer |
| HOXB4-R2 | SEQ ID NO: 17 | CCAAAATCCCGACAAACCG | HOXB4 reverse primer |
| HOXB4-P2 | SEQ ID NO: 18 | FAM-CGGTTAGAGGCGAGAGAGTAGTTT-BQ1 | HOXB4 detection probe |
| HOXB4-F3 | SEQ ID NO: 19 | CGGGTTTCGGGCGGCGCGC | HOXB4 forward primer |
| HOXB4-R3 | SEQ ID NO: 20 | CGAACGATAACGAAAACGACG | HOXB4 reverse primer |
| HOXB4-P3 | SEQ ID NO: 21 | FAM-CGTGTATCGTGTAGCGTTACGCGG-BQ1 | HOXB4 detection probe |
| SRCIN1-F1 | SEQ ID NO: 4 | TCGTGTGTCGTCGTTCAGAC | SRCIN1 forward primer |
| SRCIN1-R1 | SEQ ID NO: 5 | GAAATACCCGCGAAAATACTG | SRCIN1 reverse primer |
| SRCIN1-P1 | SEQ ID NO: 6 | AGTTTTACGTTGGAGAAGCGTCGG | SRCIN1 detection probe |

| | | | |
|---|---|---|---|
| SRCIN1-F2 | SEQ ID NO: 22 | TATCGTGTATCGTCGTTCGGAC | SRCIN1 forward primer |
| SRCIN1-R1 | SEQ ID NO: 5 | GAAATACCCGCGAAAATACTG | SRCIN1 reverse primer |
| SRCIN1-P1 | SEQ ID NO: 6 | AGTTTTACGTTGGAGAAGCGTCGG | SRCIN1 detection probe |
| A3-TqMF | SEQ ID NO: 13 | GGAGGTTTAGTAAGTTTTTTGGATT | β-actin gene forward primer |
| A3-TqMR | SEQ ID NO: 14 | CAATAAAACCTACTCCTCCCTTA | β-actin gene reverse primer |
| A3-TqP | SEQ ID NO: 15 | FAM-TTGTGTGTTGGGTGGTGGTT-BQ1 | β-actin gene detection probe |

Various solution preparation systems are consistent; the solution preparation systems and various amplification procedures are consistent, and the amplification procedures are the same as those in example 2.

Detection results are shown in Table 10 and Table 11.

**Table 10 Detection results of HOXB4 in sputum samples (normal group vs. total cancer groups)**

| Group | Specificity | Sensitivity |
|---|---|---|
| F1, R1, P1 | 93.3% | 72.0% |
| F2, R2, P2 | 93.3% | 44.0% |
| F3, R3, P3 | 93.3% | 68.0% |

**Table 11 Detection results of SRCIN1 in sputum samples (normal group vs. total cancer groups)**

| Group | Specificity | Sensitivity |
|---|---|---|
| F1, R1, P1 | 93.3% | 56.0% |
| F2, R1, P1 | 93.3% | 52.0% |

The results show that, for different primer pairs in the same region, the detection results will be affected. In the case of consistent specificity, primer and probe combinations of HOXB-F1, HOXB-R1, HOXB-P1, SRCIN1-F1, SRCIN1-R1, and SRCIN1-P1 have higher sensitivity.

## Claims

1. A gene methylation detection kit, **characterized by** comprising:
a HOXB4 gene methylation detection reagent, and a SRC1N1 gene methylation detection reagent, wherein
the HOXB4 gene methylation detection reagent comprises:
a first primer pair, comprising:
a first forward primer comprising a nucleotide sequence having an identity of at least 85% or at least 90% or at least 91% or at least 92% or at least 93% or at least 94% or at least 95% or at least 96% or at least 97% or at least 98% or at least 99% or 100% to any one of nucleotide sequences shown in SEQ ID NO: 1, SEQ ID NO: 16, or SEQ ID NO: 19; and
a first reverse primer comprising a nucleotide sequence having an identity of at least 85% or at least 90% or at least 91% or at least 92% or at least 93% or at least 94% or at least 95% or at least 96% or at least 97% or at least 98% or at least 99% or 100% to any one of nucleotide sequences shown in SEQ ID NO: 2, SEQ ID NO: 17, or SEQ ID NO: 20;
or
a first probe, comprising a nucleotide sequence having an identity of at least 85% or at least 90% or at least 91% or at least 92% or at least 93% or at least 94% or at least 95% or at least 96% or at least 97% or at least 98% or at least 99% or 100% to any one of nucleotide sequences shown in SEQ ID NO: 3, SEQ ID NO: 18, SEQ ID NO: 21, or a complementary sequence thereof; and
the SRC1N1 gene methylation detection reagent comprises:
a second primer pair, comprising:
a second forward primer comprising a nucleotide sequence having an identity of at least 85% or at least 90% or at least 91% or at least 92% or at least 93% or at least 94% or at least 95% or at least 96% or at least 97% or at least 98% or at least 99% or 100% to any one of nucleotide sequences shown in SEQ ID NO: 4 or SEQ ID NO: 22; and
a second reverse primer comprising a nucleotide sequence having an identity of at least 85% or at least 90% or at least 91% or at least 92% or at least 93% or at least 94% or at least 95% or at least 96% or at least 97% or at least 98% or at least 99% or 100% to a nucleotide sequence shown in SEQ ID NO: 5;
or
a second probe for methylation detection of the SRC1N1 gene, comprising a nucleotide sequence having an identity of at least 85% or at least 90% or at least 91% or at least 92% or at least 93% or at least 94% or at least 95% or at least 96% or at least 97% or at least 98% or at least 99% or 100% to a nucleotide sequence shown as SEQ ID NO: 6 or a complementary sequence thereof.

2. The kit of claim 1, **characterized in that**,
a nucleotide sequence of the first forward primer is shown in SEQ ID NO: 1, and a nucleotide sequence of the first reverse primer is shown in shown in SEQ ID NO: 2; or
a nucleotide sequence of the first forward primer is shown in SEQ ID NO: 16, and a nucleotide sequence of the first reverse primer is shown in shown in SEQ ID NO: 17; or
a nucleotide sequence of the first forward primer is shown in SEQ ID NO: 19, and a nucleotide sequence of the first reverse primer is shown in shown in SEQ ID NO: 20.

3. The kit of claim 1 or claim 2, **characterized in that**, a nucleotide sequence of the first probe is shown in SEQ ID NO: 3, SEQ ID NO: 18, SEQ ID NO: 21, or a complementary sequence thereof.

4. The kit of any one of claim 1 to 3, **characterized in that**,
a nucleotide sequence of the first forward primer is shown in SEQ ID NO: 1, and a nucleotide sequence of the first reverse primer is shown in shown in SEQ ID NO: 2; and/or
a nucleotide sequence of the first probe is shown in SEQ ID NO: 3.

5. The kit of any one of claim 1 to 4, **characterized in that**,
a nucleotide sequence of the second forward primer is shown in SEQ ID NO: 4, and a nucleotide sequence of the second reverse primer is shown in shown in SEQ ID NO: 5; or
a nucleotide sequence of the second forward primer is shown in SEQ ID NO: 22, and a nucleotide sequence of the second reverse primer is shown in shown in SEQ ID NO: 5.

6. The kit of any one of claim 1 to 5, **characterized in that**,
a nucleotide sequence of the second forward primer is shown in SEQ ID NO: 4, and a nucleotide sequence of the second reverse primer is shown in shown in SEQ ID NO: 22.

7. The kit of any one of claims 1 to 6, **characterized in that**, a nucleotide sequence of the second probe is shown in SEQ ID NO: 6 or a complementary sequence thereof, preferably SEQ ID NO: 6.

8. The kit of any one of claims 1 to 7, **characterized by** comprising the first primer pair, the first probe, the second primer pair, and the second probe.

9. Use of the kit of any one of claims 1 to 8 in detecting a lung cancer in a subject.

10. A method for detecting a lung cancer in a subject, **characterized by** comprising:
detecting methylation levels of HOXB4 and SRCIN1 genes in a sample derived from a subject, by the detection kit of any one of claims 1 to 8;
comparing methylation levels of HOXB4 and SRCIN1 genes of the sample and a normal control sample; and
detecting the lung cancer based on a deviation of the methylation levels of the sample and the normal control sample.

11. The kit of any one of claims 1 to 8 for use in a method for treating a tumor in a subject, **characterized by** the method comprising:
detecting methylation levels of HOXB4 and SRCIN1 genes in a sample from the subject, by the kit of any one of claims 1 to 8;
comparing the methylation levels of HOXB4 and SRCIN1 genes of the sample and a normal control sample;
diagnosing the lung cancer based on deviation of the methylation levels of the sample and the normal control sample; and
administering a drug for lung cancer to the subject.

12. The method of claim 10 or the kit of claim 11, **characterized in that** the methylation levels of the HOXB4 and SRCIN1 genes are detected by using quantitative methylation specific PCR.

13. The method or the kit of any one of claim 10 to 12, **characterized in that** the sample is at least one selected from a group consisting of pulmonary alveoli lavage fluid, tissues, hydrothorax, sputum, blood, serum, plasma, urine, prostatic fluid, and excrements, preferably the sample is pulmonary alveoli lavage fluid or sputum.

14. The use, the method or the kit of any one of claims 9 to 13, **characterized in that** the lung cancer is small cell lung cancer or non-small cell lung cancer.

15. The use, the method of any one of claims 9 to 14, **characterized in that** the lung cancer is squamous-cell carcinoma or adenomatous carcinoma.

## Patentansprüche

1. Kit zum Nachweis der Genmethylierung, **dadurch gekennzeichnet, dass** es Folgendes umfasst:
ein Reagenz zum Nachweis der HOXB4-Genmethylierung und ein Reagenz zum Nachweis der SRC1N1-Genmethylierung, wobei
das Reagenz zum Nachweis der HOXB4-Genmethylierung Folgendes umfasst:
ein erstes Primerpaar, umfassend:
einen ersten Vorwärtsprimer, umfassend eine Nukleotidsequenz mit einer Identität von mindestens 85 % oder mindestens 90 % oder mindestens 91 % oder mindestens 92 % oder mindestens 93 % oder mindestens 94 % oder mindestens 95 % oder mindestens 96 % oder mindestens 97 % oder mindestens 98 % oder mindestens 99 % oder 100 % mit einer beliebigen der in SEQ ID NO: 1, SEQ ID NO: 16 oder SEQ ID NO: 19 dargestellten Nukleotidsequenzen; und
einen ersten Rückwärtsprimer, umfassend eine Nukleotidsequenz mit einer Identität von mindestens 85 % oder mindestens 90 % oder mindestens 91 % oder mindestens 92 % oder mindestens 93 % oder mindestens 94 % oder mindestens 95 % oder mindestens 96 % oder mindestens 97 % oder mindestens 98 % oder mindestens 99 % oder 100 % mit einer beliebigen der in SEQ ID NO: 2, SEQ ID NO: 17 oder SEQ ID NO: 20 dargestellten Nukleotidsequenzen;
oder
eine erste Sonde, umfassend eine Nukleotidsequenz mit einer Identität von mindestens 85 % oder mindestens 90 % oder mindestens 91 % oder mindestens 92 % oder mindestens 93 % oder mindestens 94 % oder mindestens 95 % oder mindestens 96 % oder mindestens 97 % oder mindestens 98 % oder mindestens 99 % oder 100 % mit einer beliebigen der in SEQ ID NO: 3, SEQ ID NO: 18, SEQ ID NO: 21 dargestellten Nukleotidsequenzen oder einer komplementären Sequenz davon; und das Reagenz zum Nachweis der SRC1N1-Genmethylierung Folgendes umfasst:
ein zweites Primerpaar, umfassend:
einen zweiten Vorwärtsprimer, umfassend eine Nukleotidsequenz mit einer Identität von mindestens 85 % oder mindestens 90 % oder mindestens 91 % oder mindestens 92 % oder mindestens 93 % oder mindestens 94 % oder mindestens 95 % oder mindestens 96 % oder mindestens 97 % oder mindestens 98 % oder mindestens 99 % oder 100 % mit einer beliebigen der in SEQ ID NO: 4 oder SEQ ID NO: 22 dargestellten Nukleotidsequenzen; und
einen zweiten Rückwärtsprimer, umfassend eine Nukleotidsequenz mit einer Identität von mindestens 85 % oder mindestens 90 % oder mindestens 91 % oder mindestens 92 % oder mindestens 93 % oder mindestens 94 % oder mindestens 95 % oder mindestens 96 % oder mindestens 97 % oder mindestens 98 % oder mindestens 99 % oder 100 % mit einer in SEQ ID NO: 5 dargestellten Nukleotidsequenz;
oder
eine zweite Sonde zum Nachweis der Methylierung des SRC1N1-Gens, umfassend eine Nukleotidsequenz mit einer Identität von mindestens 85 % oder mindestens 90 % oder mindestens 91 % oder mindestens 92 % oder mindestens 93 % oder mindestens 94 % oder mindestens 95 % oder mindestens 96 % oder mindestens 97 % oder mindestens 98 % oder mindestens 99 % oder 100 % mit einer als SEQ ID NO: 6 dargestellten Nukleotidsequenz oder einer komplementären Sequenz davon.

2. Kit nach Anspruch 1, **dadurch gekennzeichnet, dass**
eine Nukleotidsequenz des ersten Vorwärtsprimers in SEQ ID NO: 1 dargestellt ist und eine Nukleotidsequenz des ersten Rückwärtsprimers in SEQ ID NO: 2 dargestellt ist; oder
eine Nukleotidsequenz des ersten Vorwärtsprimers in SEQ ID NO: 16 dargestellt ist und eine Nukleotidsequenz des ersten Rückwärtsprimers in SEQ ID NO: 17 dargestellt ist; oder
eine Nukleotidsequenz des ersten Vorwärtsprimers in SEQ ID NO: 19 dargestellt ist und eine Nukleotidsequenz des ersten Rückwärtsprimers in SEQ ID NO: 20 dargestellt ist.

3. Kit nach Anspruch 1 oder Anspruch 2, **dadurch gekennzeichnet, dass** eine Nukleotidsequenz der ersten Sonde in SEQ ID NO: 3, SEQ ID NO: 18, SEQ ID NO: 21 oder einer komplementären Sequenz davon dargestellt ist.

4. Kit nach einem der Ansprüche 1 bis 3, **dadurch gekennzeichnet, dass**
eine Nukleotidsequenz des ersten Vorwärtsprimers in SEQ ID NO: 1 dargestellt ist und eine Nukleotidsequenz des ersten Rückwärtsprimers in SEQ ID NO: 2 dargestellt ist; und/oder
eine Nukleotidsequenz der ersten Sonde in SEQ ID NO: 3 dargestellt ist.

5. Kit nach einem der Ansprüche 1 bis 4, **dadurch gekennzeichnet, dass**
eine Nukleotidsequenz des zweiten Vorwärtsprimers in SEQ ID NO: 4 dargestellt ist und eine Nukleotidsequenz des zweiten Rückwärtsprimers in SEQ ID NO: 5 dargestellt ist; oder
eine Nukleotidsequenz des zweiten Vorwärtsprimers in SEQ ID NO: 22 dargestellt ist und eine Nukleotidsequenz des zweiten Rückwärtsprimers in SEQ ID NO: 5 dargestellt ist.

6. Kit nach einem der Ansprüche 1 bis 5, **dadurch gekennzeichnet, dass**
eine Nukleotidsequenz des zweiten Vorwärtsprimers in SEQ ID NO: 4 dargestellt ist und eine Nukleotidsequenz des zweiten Rückwärtsprimers in SEQ ID NO: 22 dargestellt ist.

7. Kit nach einem der Ansprüche 1 bis 6, **dadurch gekennzeichnet, dass** eine Nukleotidsequenz der zweiten Sonde in SEQ ID NO: 6 oder einer komplementären Sequenz davon, vorzugsweise SEQ ID NO: 6, dargestellt ist.

8. Kit nach einem der Ansprüche 1 bis 7, **dadurch gekennzeichnet, dass** es das erste Primerpaar, die erste Sonde, das zweite Primerpaar und die zweite Sonde umfasst.

9. Verwendung des Kits nach einem der Ansprüche 1 bis 8 zum Nachweis eines Lungenkrebses in einem Subjekt.

10. Verfahren zum Nachweis eines Lungenkrebses in einem Subjekt, **dadurch gekennzeichnet, dass** es Folgendes umfasst:
Ermitteln der Methylierungsgrade der HOXB4- und SRCIN1-Gene in einer von einem Subjekt stammenden Probe mittels des Nachweiskits nach einem der Ansprüche 1 bis 8;
Vergleich der Methylierungsgrade der HOXB4- und SRCIN1-Gene der Probe mit denen einer normalen Kontrollprobe; und
Nachweis des Lungenkrebses auf der Grundlage einer Abweichung der Methylierungsgrade der Probe und der normalen Kontrollprobe.

11. Kit nach einem der Ansprüche 1 bis 8 zur Verwendung in einem Verfahren zur Behandlung eines Tumors in einem Subjekt, **dadurch gekennzeichnet, dass** das Verfahren Folgendes umfasst:
Nachweis der Methylierungsgrade der HOXB4- und SRCIN1-Gene in einer von einem Subjekt stammenden Probe mittels des Nachweiskits nach einem der Ansprüche 1 bis 8;
Vergleich der Methylierungsgrade der HOXB4- und SRCIN1-Gene der Probe mit denen einer normalen Kontrollprobe;
Diagnostizieren des Lungenkrebses auf der Grundlage der Abweichung der Methylierungsgrade der Probe und der normalen Kontrollprobe; und
Verabreichung eines Arzneimittels gegen Lungenkrebs an das Subjekt.

12. Verfahren nach Anspruch 10 oder Kit nach Anspruch 11, **dadurch gekennzeichnet, dass** die Methylierungsgrade der HOXB4- und SRCIN1-Gene unter Verwendung einer quantitativen methylierungsspezifischen PCR nachgewiesen werden.

13. Verfahren oder Kit nach einem der Ansprüche 10 bis 12, **dadurch gekennzeichnet, dass** die Probe mindestens eine ist, die ausgewählt ist aus einer Gruppe bestehend aus bronchoalveolärer Lavageflüssigkeit der Lungenalveolen, Geweben, Hydrothorax, Sputum, Blut, Serum, Plasma, Urin, Prostataflüssigkeit und Exkrementen, wobei die Probe vorzugsweise bronchoalveoläre Lavageflüssigkeit der Lungenalveolen oder Sputum ist.

14. Verwendung, Verfahren oder Kit nach einem der Ansprüche 9 bis 13, **dadurch gekennzeichnet, dass** der Lungenkrebs ein kleinzelliger Lungenkrebs oder ein nichtkleinzelliger Lungenkrebs ist.

15. Verwendung oder Verfahren nach einem der Ansprüche 9 bis 14, **dadurch gekennzeichnet, dass** der Lungenkrebs ein Plattenepithelkarzinom oder ein adenomatöses Karzinom ist.

## Revendications

1. Kit de détection de méthylation génique, **caractérisé en ce qu'**il comprend :
un réactif de détection de méthylation du gène HOXB4 et un réactif de détection de méthylation du gène SRC1N1 ;
ledit réactif de détection de méthylation du gène HOXB4 comprenant :
une première paire d'amorces, comprenant :
une première amorce sens comprenant une séquence nucléotidique ayant au moins 85 %, au moins 90 %, au moins 91 %, au moins 92 %, au moins 93 %, au moins 94 %, au moins 95 %, au moins 96 %, au moins 97 %, au moins 98 %, au moins 99 % ou 100 % d'identité avec l'une quelconque des séquences nucléotidiques présentées dans la SEQ ID N° : 1, la SEQ ID N° : 16 ou la SEQ ID N° : 19, et
une première amorce anti-sens comprenant une séquence nucléotidique ayant au moins 85 %, au moins 90 %, au moins 91 %, au moins 92 %, au moins 93 %, au moins 94 %, au moins 95 %, au moins 96 %, au moins 97 %, au moins 98 %, au moins 99 % ou 100 % d'identité avec l'une quelconque des séquences nucléotidiques présentées dans la SEQ ID N° : 2, la SEQ ID N° : 17 ou la SEQ ID N° : 20,
ou
une première sonde comprenant une séquence nucléotidique ayant au moins 85 %, au moins 90 %, au moins 91 %, au moins 92 %, au moins 93 %, au moins 94 %, au moins 95 %, au moins 96 %, au moins 97 %, au moins 98 %, au moins 99 % ou 100 % d'identité avec l'une quelconque des séquences nucléotidiques présentées dans la SEQ ID N° : 3, la SEQ ID N° : 18, la SEQ ID N° : 21, ou avec une séquence complémentaire de celles-ci, et
ledit réactif de détection de méthylation du gène SRC1N1 comprenant :
une deuxième paire d'amorces, comprenant :
une deuxième amorce sens comprenant une séquence nucléotidique ayant au moins 85 %, au moins 90 %, au moins 91 %, au moins 92 %, au moins 93 %, au moins 94 %, au moins 95 %, au moins 96 %, au moins 97 %, au moins 98 %, au moins 99 % ou 100 % d'identité avec l'une quelconque des séquences nucléotidiques présentées dans la SEQ ID N° : 4 ou la SEQ ID N° : 22, et
une deuxième amorce anti-sens comprenant une séquence nucléotidique ayant au moins 85 %, au moins 90 %, au moins 91 %, au moins 92 %, au moins 93 %, au moins 94 %, au moins 95 %, au moins 96 %, au moins 97 %, au moins 98 %, au moins 99 % ou 100 % d'identité avec une séquence nucléotidique présentée dans la SEQ ID N° : 5,
ou
une deuxième sonde pour la détection de méthylation du gène SRC1N1, comprenant une séquence nucléotidique ayant au moins 85 %, au moins 90 %, au moins 91 %, au moins 92 %, au moins 93 %, au moins 94 %, au moins 95 %, au moins 96 %, au moins 97 %, au moins 98 %, au moins 99 %, ou 100 % d'identité avec une séquence nucléotidique présentée dans la SEQ ID N° : 6 ou avec une séquence complémentaire de celle-ci.

2. Kit selon la revendication 1, **caractérisé en ce que** :
une séquence nucléotidique de la première amorce sens est présentée dans la SEQ ID N° : 1 et une séquence nucléotidique de la première amorce anti-sens est présentée dans la SEQ ID N° : 2, ou
une séquence nucléotidique de la première amorce sens est présentée dans la SEQ ID N° : 16 et une séquence nucléotidique de la première amorce anti-sens est présentée dans la SEQ ID N° : 17, ou
une séquence nucléotidique de la première amorce sens est présentée dans la SEQ ID N° : 19 et une séquence nucléotidique de la première amorce anti-sens est présentée dans la SEQ ID N° : 20.

3. Kit selon la revendication 1 ou la revendication 2, **caractérisé en ce que** la séquence nucléotidique de la première sonde est présentée dans la SEQ ID N° : 3, la SEQ ID N° : 18, la SEQ ID N° : 21 ou une séquence complémentaire de celles-ci.

4. Kit selon l'une quelconque des revendications 1 à 3, **caractérisé en ce que** :
une séquence nucléotidique de la première amorce sens est présentée dans la SEQ ID N° : 1 et une séquence nucléotidique de la première amorce anti-sens est présentée dans la SEQ ID N° : 2, et/ou
une séquence nucléotidique de la première sonde est présentée dans la SEQ ID N° : 3.

5. Kit selon l'une quelconque des revendications 1 à 4, **caractérisé en ce que** :
une séquence nucléotidique de la deuxième amorce sens est présentée dans la SEQ ID N° : 4 et une séquence nucléotidique de la deuxième amorce anti-sens est présentée dans la SEQ ID N° : 5, ou
une séquence nucléotidique de la deuxième amorce sens est présentée dans la SEQ ID N° : 22 et une séquence nucléotidique de la deuxième amorce anti-sens est présentée dans la SEQ ID N° : 5.

6. Kit selon l'une quelconque des revendications 1 à 5, **caractérisé en ce que** :
une séquence nucléotidique de la deuxième amorce sens est présentée dans la SEQ ID N° : 4 et une séquence nucléotidique de la deuxième amorce anti-sens est présentée dans la SEQ ID N° : 22.

7. Kit selon l'une quelconque des revendications 1 à 6, **caractérisé en ce qu'**une séquence nucléotidique de la deuxième sonde est présentée dans la SEQ ID N° : 6 ou une séquence complémentaire de celle-ci, de préférence dans la SEQ ID N° : 6.

8. Kit selon l'une quelconque des revendications 1 à 7, **caractérisé en ce qu'**il comprend la première paire d'amorces, la première sonde, la deuxième paire d'amorces et la deuxième sonde.

9. Utilisation du kit selon l'une quelconque des revendications 1 à 8 pour détecter un cancer du poumon chez un sujet.

10. Méthode de détection d'un cancer du poumon chez un sujet, **caractérisée en ce qu'**elle comprend :
la détection du niveau de méthylation des gènes HOXB4 et SRCIN1 dans un échantillon provenant d'un sujet, au moyen du kit de détection selon l'une quelconque des revendications 1 à 8,
la comparaison du niveau de méthylation des gènes HOXB4 et SRCIN1 de l'échantillon à celui d'un échantillon témoin normal, et
la détection du cancer du poumon en fonction d'un écart entre le niveau de méthylation de l'échantillon et celui de l'échantillon témoin normal.

11. Kit selon l'une quelconque des revendications 1 à 8, destiné à une utilisation dans une méthode de traitement d'une tumeur chez un sujet, **caractérisé en ce que** la méthode comprend :
la détection du niveau de méthylation des gènes HOXB4 et SRCIN1 dans un échantillon provenant du sujet, au moyen du kit selon l'une quelconque des revendications 1 à 8,
la comparaison du niveau de méthylation des gènes HOXB4 et SRCIN1 de l'échantillon à celui d'un échantillon témoin normal,
le diagnostic du cancer du poumon en fonction d'un écart entre le niveau de méthylation de l'échantillon et celui de l'échantillon témoin normal, et
l'administration d'un médicament contre le cancer du poumon au sujet.

12. Méthode selon la revendication 10 ou kit selon la revendication 11, **caractérisés en ce que** le niveau de méthylation des gènes HOXB4 et SRCIN1 est détecté au moyen d'une PCR quantitative spécifique à la méthylation.

13. Méthode ou kit selon l'une quelconque des revendications 10 à 12, **caractérisés en ce que** l'échantillon est au moins un échantillon choisi dans un groupe comprenant le liquide de lavage alvéolaire pulmonaire, les tissus, l'hydrothorax, les expectorations, le sang, le sérum, le plasma, l'urine, le liquide prostatique et les excréments ; l'échantillon consistant de préférence en du liquide de lavage alvéolaire pulmonaire ou des expectorations.

14. Utilisation, méthode ou kit selon l'une quelconque des revendications 9 à 13, **caractérisés en ce que** le cancer du poumon est un cancer du poumon à petites cellules ou un cancer du poumon non à petites cellules.

15. Utilisation, méthode selon l'une quelconque des revendications 9 à 14, **caractérisée en ce que** le cancer du poumon est un carcinome épidermoïde ou un carcinome adénomateux.
